# EUROPEAN PATENT APPLICATION

(11) **EP 4 781 939 A1**
(43) Date of publication of application: **29.07.2026**
(21) Application number: 25153759.3
(22) Date of filing: 24.01.2025
(51) Int. Cl.: A61B 18/20

(54) **PERSONAL CARE DEVICE WITH MOVEMENT TRACKING**

(71) Applicant: Koninklijke Philips N.V., 5656 AE Eindhoven (NL)
(72) Inventor: FLINSENBERG, Ingrid Christina Maria, 5656 AG Eindhoven (NL); KINGMA, Harmen Andries, 5656 AG Eindhoven (NL); VAN ZUTPHEN, Martijn, 5656 AG Eindhoven (NL)
(74) Representative: Philips Intellectual Property & Standards

(57) **Abstract**

A personal care device is for application against the skin, such as an IPL device. An optical displacement sensor generates a displacement signal representative of a displacement of the device relative to the skin and a movement sensor generates a movement signal representative of an absolute movement of the device. The displacement signal is used to generate a first contribution to a movement output signal and the movement signal is used to generate a second contribution to the movement output signal. The movement output signal is generated primarily or entirely based the first contribution when the displacement signal is determined to be reliable, and primarily or entirely based on the second contribution when the displacement signal is determined to be not reliable. In this way, relative displacement sensing and absolute motion sensing are combined to accurately track movement of the device.

## Description

### FIELD OF THE INVENTION

This invention relates to personal care devices which incorporate movement tracking of the device.

### BACKGROUND OF THE INVENTION

It is well known to track the movement of a personal care device in order to provide automated guidance to a user or to automatically control operation of the device. For example, guidance can be used to assist a user when brushing teeth, shaving, using an epilator etc.

For example, light-based epilators are well-known. People use hair epilators or depilators to remove unwanted hair. Typical target areas for women are the face, armpit, arm, leg, and bikini line. Men use light-based epilators on the chest and back.

Light-based hair removal devices are based on the filtered output of a flashlamp, known as Intense Pulsed Light or IPL. IPL devices also have the advantage that they enable various skin treatments to be combined with a hair removal mode.

It is known to provide an IPL device with an associated app, which is used to provide coaching features. In one approach, a particular number of flashes of IPL light is assumed, regardless of leg length. In another approach, during first-time use of the IPL device, the app asks the user to measure the length of their body part in question (e.g., the leg) and then calculates the required number of flashes (based on the treatment window width). After this, the app asks the user to count the actual number of flashes for one stroke, and then reports a coverage percentage.

This coaching approach is not considered very "smart" since it involves user input to calculate coverage. It requires a certain effort by the user (like getting a measuring tape) which will discourage many users.

Smarter executions of the length-measuring and flash counting approach have been proposed. For example, it is possible to measure the length of the leg using a photo, and to automatically count flashes using analysis of the sound that the device makes.

It would be of interest to users to enable an automatic step size measurement to be implemented using sensors. Measuring the actual step size between each flash (or averaged over a few flashes) using sensors would allow coaching concepts which require less user interaction and would enable more granular feedback regarding the performance over the length of the treatment strokes.

The applicant has proposed the use of a built-in movement sensor, such as a 6-axis IMU (3-axis accelerometer, 3-axis gyroscope) to calculate the average step size based on the IMU data, and behavioral patterns used to coach the user. This allows the user to follow their normal routine as long as he/she adheres to the required behavioral patterns.

However, because the IMU measures movements of the device, and not only the movements relative to the skin, the user needs to adhere to certain behavioral patterns to be able to provide accurate coaching on step-size.

The step-size can only be computed after the step has been made. This means that coaching the user to stop at a certain point because the right distance has been traversed (e.g. a window size distance or an integer multiple of window sizes) is not possible.

If the user makes too large steps and hence does not treat a certain area of the skin, it is consequently not possible to guide the user to return to a missed spot to correct the mistake. This could require multiple iterations to guide the user back to a missed spot (again because the step-size is only computed after the step movement). These multiple iterations, as well as their combination, would need to be very accurate to prevent overlap. That accuracy is not currently possible to achieve.

There is a need for an improved motion tracking system for personal care devices, in order to enable improved coaching and/or to enable automated operation of the personal care device with a reduced need for user interaction.

### SUMMARY OF THE INVENTION

The invention is defined by the claims.

According to examples in accordance with an aspect of the invention, there is provided a personal care device for application against the skin, comprising:
an optical displacement sensor configured to generate a displacement signal representative of a displacement of the device relative to the skin;
a movement sensor configured to generate a movement signal representative of an absolute movement of the device;
a controller configured to generate a movement output signal representative of a movement of the device,
wherein the controller is configured to:
   receive the displacement signal and the movement signal;
   use the displacement signal to generate a first contribution to the movement output signal and use the movement signal to generate a second contribution to the movement output signal;
   determine a reliability of the displacement signal; and
   generate the movement output signal based more, or entirely, on the first contribution when the displacement signal is determined to be reliable and based more, or entirely, on the second contribution when the displacement signal is determined to be not reliable.

This device tracks its position over the skin primarily by using optical displacement tracking when a reliable signal is generated (for example while contact with the skin is maintained), and primarily by using absolute motion tracking when a reliable displacement signal is not available (for example when contact with the skin is lost). This provides accurate motion tracking, so that operation of the device can be controlled in the most effective manner and/or most suitable user feedback can be provided.

For some optical displacement sensors, skin contact is needed for a reliable signal, so that as soon as skin contact is lost, the movement signal is used. For other optical displacement sensors, their output can be used as long as there is a limited distance, so that the movement signal only needs to be used when a spacing exceeds a threshold at which the displacement signal cannot be used to track device movement.

Thus, the reliability of the displacement signal may be determined by different approaches depending on the design of the optical displacement sensor.

The device thus preferably comprises a skin contact sensor configured to generate a skin contact signal representative of the device being in contact with the skin. Optionally, the controller may then be configured to determine that the displacement signal is not reliable when the device is not in contact with the skin, for example, when skin contact is lost. The skin contact sensor for example comprises a capacitance sensor.

Alternatively, the device may comprise a skin distance sensor adapted to detect a distance between the device and the skin. The controller may be configured to determine that the displacement signal is not reliable when the distances exceeds a threshold.

Alternatively, the displacement sensor may have its own built-in method for determining if the displacement signal is reliable. For example, the displacement sensor may generate an output indicating the quality. For example, the reliability is based on a measure of contrast as detected by the optical displacement sensor. For example, the reliability is based on generated output of the displacement sensor.

The movement sensor for example comprises a 6-axis inertial monitoring unit, IMU.

The personal care device for example comprises an IPL device for delivering IPL light to a user's skin during IPL treatment, and comprises:
an IPL delivery unit comprising:
   a light source; and
   a light delivery window;
wherein the controller is configured to control the light source to deliver IPL light to the light delivery window in dependence on the movement output signal.

The movement tracking may thus be used to control when light flashes are delivered by an IPL delivery unit. This can be used to ensure there is uniform and uninterrupted coverage of the skin area to be treated while avoiding over exposure.

The controller may be configured to determine a position of the IPL device over time by adding displacements during sensor sampling windows. The motion tracking thus derives incremental motion steps. For example, the controller determines a displacement of the device during a first sensor sampling window. Then, the controller determines a displacement of the device during a second sensor sampling window. The controller determines the position of the IPL device by adding the displacements during the first sensor sampling window and the second sensor sampling window.

The controller is, for example, configured, when the displacement signal is reliable (e.g., during times of skin contact), to compensate for drift of the movement sensor by using the displacement signal.

This means the movement sensor estimates based on the movement signal are more reliable in case the displacement signal becomes unreliable (e.g., when skin contact is lost). If the duration of loss of skin contact is sufficiently short, the quality of the estimates based on the movement signal is generally sufficiently high so these estimates can be used to maintain tracking.

Similarly, the movement signal may be used to enhance the accuracy of the displacement signal, for example, by providing an estimated displacement signal during times when the displacement signal is unreliable.

The displacement sensor, for example, may comprise a non-contact optical displacement sensor. In this case, the distance sensor mentioned above may be employed. The displacement signal can still be used even if skin contact is lost to a limited extent (i.e., a spacing to the skin is below a threshold such that signal compensation can be applied).

The distance sensor for example comprises a IMU sensor. This can be used to track the amount of movement away from the skin. This IMU sensor is, for example, the same sensor as the movement sensor.

The controller may comprise an AI model configured to estimate a displacement of the IPL device from a previous location of the IPL delivery unit. Thus, AI may be used to perform the motion tracking. In this case, a more complex combination of the first and second contributions to derive the movement output signal may be implemented by the AI model. For example, the AI model is configured to generate the movement output signal based on the displacement signal and the movement signal.

The controller may be configured to generate a quality factor that indicates an accuracy of the displacement estimate, and the controller is configured to control the light source to deliver a light flash only when the quality factor exceeds a threshold.

In this way, the light flash is only delivered when the motion tracking is determined to be sufficiently reliable that the determined position can be relied upon.

The controller is for example configured to control the light source to deliver IPL light when a current position of the IPL delivery unit is at a distance from the positions of a set of (e.g., all) previous deliveries of IPL light that is at least equal to a dimension of the light delivery window (e.g. the size in a dominant axis direction). This avoids double exposure of skin areas. The dominant axis direction is the direction in which the device is being moved over the skin, to perform a line of treatments or to move between lines of treatment.

In another example, the controller may be configured to deliver a flash of IPL light when a current position of the IPL delivery unit is at a distance from the positions of a set of (e.g., all) previous deliveries of IPL light that is approximately equal to an integer multiple of the dimension of the light delivery window. By requiring a spacing of multiple light delivery window dimensions, the user can retreat to fill in gaps that have not yet been treated, without double flashing previous areas with IPL light.

The controller may be configured to determine if the IPL delivery unit has been rotated using the movement signal, and if the IPL delivery unit has been rotated, to reset stored locations of previous deliveries of light flashes. If the device has been rotated, the motion tracking using skin contact sensing may become unreliable so that the previously stored positions are reset.

The system may further comprise an output interface, and the controller is configured to control the output interface to provide user feedback in respect of the correct amount of movement between flashes to achieve correct skin coverage. This correct amount of movement is movement of at least the dimension of the light output window. The correct skin coverage avoids large gaps between treated areas as well as avoiding multiple exposures at the same location. The feedback can also be aimed at guiding the user to not exceed a maximum movement speed, thus avoiding that the flash electronics have not been fully recharged when a flash should occur.

The invention also provides a method of tracking motion of a personal care device that is for application against the skin, comprising:
receiving a displacement signal representative of a displacement of the device relative to the skin;
receiving a movement signal representative of an absolute movement of the device;
generating a movement output signal representative of a movement of the device by:
   using the displacement signal to generate a first contribution to a movement output and using the movement signal to generate a second contribution to the movement output signal;
   determining a reliability of the displacement signal; and
   generating the movement output signal based more, or entirely, on the first contribution when the displacement signal is determined to be reliable and based more, or entirely, on the second contribution when the displacement signal is determined to be not reliable.

The invention also provides a method of controlling IPL device for delivering IPL light to a user's skin during an IPL treatment, comprising:
tracking motion of an IPL delivery unit of the IPL device using the method described above; and
controlling a light source of the IPL device to deliver light flashes to the light delivery window, in dependence on the tracked motion of the IPL delivery unit.

The invention also provides a computer program comprising computer program code which is adapted, when said program is run on a computer, to implement the method described above.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an IPL device;
Fig. 2 shows an IPL system combining a hand-held device and remote device for remote processing and/or feedback;
Fig. 3 shows a method of controlling the device of Fig. 1 or 2; and
Fig. 4 shows how the motion tracking is performed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a personal care device for application against the skin, such as an IPL device. An optical displacement sensor generates a displacement signal representative of a displacement of the device relative to the skin and a movement sensor generates a movement signal representative of an absolute movement of the device. The displacement signal is used to generate a first contribution to a movement output signal and the movement signal is used to generate a second contribution to the movement output signal. The movement output signal is generated primarily or entirely based the first contribution when the displacement signal is determined to be reliable, and primarily or entirely based on the second contribution when the displacement signal is determined to be not reliable. In this way, relative displacement sensing and absolute motion sensing are combined to accurately track movement of the device.

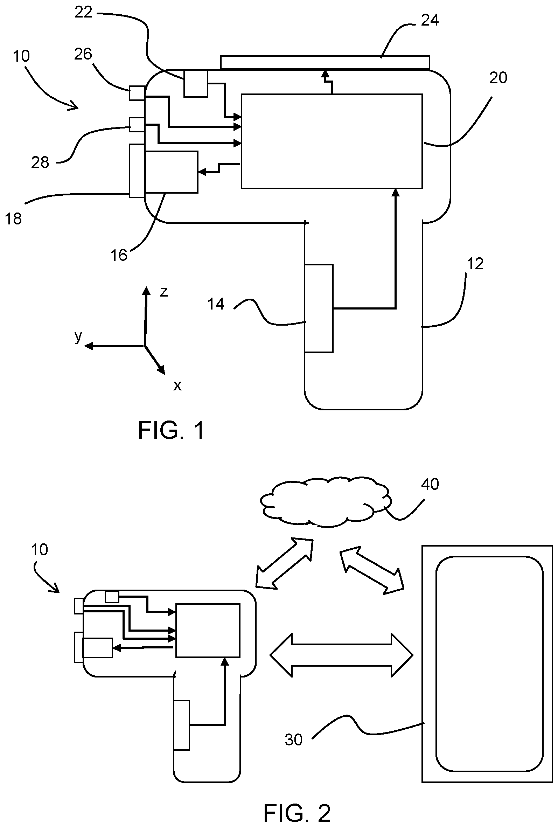

Fig. 1 shows an IPL device 10, in the form of a hand-held device having a handle 12 with a trigger 14, a light source 16 providing light through a light delivery window 18. A controller 20 controls the light source 16 to deliver light flashes of IPL light to the light delivery window 18.

The controller 20 also reports the timing of the light flashes (as "light flash timing"). This light flash timing may instead or additionally be derived from activation of the trigger 14.

An optical displacement sensor 26 is used to generate a displacement signal representative of a displacement of the device 10 relative to the skin. A movement sensor 22 senses the absolute motion (i.e., within 3D space) of the IPL device 10.

The movement sensor 22, for example, comprises a 6- axis Inertial Monitoring Unit, IMU. A 6-axis IMU measures 3-axis accelerations and 3-axis gyroscope signals. The IMU is a small surface mount device (SMD) component which can be soldered directly to the printed circuit board (PCB) of the device 10. Movements measured at the location of the IMU can be translated to movements at the treatment location using a coordinate transformation.

Inertial Measurement Units (IMUs) are used in many situations where movement of devices must be detected/measured. For instance, a step counter in a smart watch, or pick-up detection in a smartphone.

The monitoring of movement between flashes using the IMU gives a low-cost implementation, since the IMU can be implemented in the handle of the device without needing to be in the head that makes contact with the skin. In this way, the same sensor may be used when different attachments are selected. The IPL device may come with several types of attachments for different body areas.

However, calculating device location based on 3D acceleration data from the IMU is not self-evident. There are several challenges to overcome:
For example, the IMU measures acceleration (in m/s²), but not position/displacement (in mm). It is possible to integrate acceleration twice to derive a distance travelled, but (inexpensive) IMUs have a noisy nature and usually have an offset/bias. This leads to inaccurate distance estimations, especially when measured over longer time durations. Measuring several seconds may already be considered a long duration.

The acceleration that the IMU measures (in 3 directions) may also contain the gravity vector, which is very dominant compared to the low "real" accelerations caused by movements of the device 10. The gravity vector can be removed using a low pass filter, but this introduces lag. Smarter methods for removing the gravity vector use the gyroscope to detect rotations, which suffer less from the mentioned lag problem.

The movement sensor 22 measures device displacement, but not the movement relative to the body. If, during the movement of the device over the body e.g., leg, the user also moves the leg itself, this leg movement cannot be measured. The system will measure the sum of both the device movement and the leg movement. Using only the movement signal, it is (in principle) impossible to discriminate between these two movements.

In the system of this disclosure, in addition to the movement sensor 22, the optical displacement sensor 26 is used to generate a displacement signal representative of a displacement of the device 10 relative to the skin.

The optical displacement sensor is preferably as close as possible to the treatment location and preferably has a field of view which is entirely occupied by the skin surface. Accidental viewing of the free space away from the skin surface should be avoided, for example when the device is rotated. Additionally, doming of the skin should be prevented. Finally, the optical displacement sensor is preferably adapted to measure along the two axes of the treatment window, i.e., both along the x-axis and the z-axis as shown in FIG. 1.

A skin contact sensor 28 is provided to detect skin contact. The skin contact sensor 28 may comprise a single sensor, or two or more sensors to detect that the light output window is flat against the skin. The skin contact sensor is for example implemented as one or more capacitive touch sensors.

In the arrangement of this disclosure, the optical displacement sensor functions as the primary movement sensor, and the movement sensor functions as a secondary movement sensor to aid during times that tracking using the primary movement sensor is lost, either because of loss of contact for a contact optical displacement sensor (which only measures when there is contact with a surface), or because of moving out of range of the sensor for a non-contact optical displacement sensor (which measures at a distance). These contact conditions indicate that the displacement signal is not reliable. Other factors may be taken into account in determining that the displacement signal is not reliable.

The displacement sensor generates a displacement signal, and this is used to generate a first contribution to a movement output signal. The movement sensor generates a movement signal and this is used to generate a second contribution to the movement output signal,
The movement output signal is generated primarily based on one or other of the first and second contributions. In particular, if the displacement signal is reliable, the first contribution is the primary contributor to the movement output signal. For example, the movement output signal is derived solely from the first contribution. If the displacement signal is not reliable, the second contribution is the primary contributor to the movement output signal. For example, the movement output signal is derived solely from the second contribution.

However, in a more complex implementation, the first and second contributions may be combined in a less binary manner. This may be the result of an implementation of the system using an AI algorithm. For example, the correct position may be determined by a trained AI algorithm from first and second contributions using ground truth data comprising the true device change in position corresponding to those first and second contributions.

In another example, a reliability of the movement signal as well as a reliability of displacement signal may be determined. The first contribution and second contribution may be combined in a manner that depends on both reliability measures.

The controller is used to process the displacement sensor signal and movement sensor signal and to control the light flash timing. In the example of Fig. 1, the controller is shown as a single entity 20 which performs all processing and control functions. For example, the controller 20 comprises a microcontroller.

However, as will be clear from Fig. 2, control of the light source and data processing or the displacement signal and the movement signal may be split between two or more different entities. For example, the controller 20 is adapted to cooperate with the two or more different entities. For example, the controller 20 comprises a local processor in the IPL device 10 that may have insufficient processing power to incorporate a machine learning algorithm, so that more processing power is needed than an embedded microcontroller (e.g., ARM-based) can typically deliver. The controller 20 is, for example, adapted to make use of more processing power externally of the IPL device 10, as is for example, further described with respect to Fig. 2.

The device of Fig. 1 also has an output interface 24, such as a screen and/or loudspeaker. The output interface 24 to the user may instead or additionally be provided by a remote device with which the hand-held device communicates as also shown in Fig. 2.

In use of known devices, a user applies the light output window to the skin and presses the trigger 14 to deliver a pulse of light. The user then slides the device over the skin to the next treatment location, and applies the trigger to deliver the next flash of light. It is also possible in some known devices to keep the trigger depressed during the line treatment. It will then automatically flash every time a capacitor in the device is charged enough, which is typically 1-2 seconds depending on the setting. The user may then just move the device to the next spot, wait for the next flash, and move on again. A line of flashes is applied along the length of a body part, before a next line is started. This line extends along a main axis direction of the light delivery window.

The conventional use of this type of device is not totally straightforward since the flashes of light should be applied in a continuous line (to avoid untreated gaps) and in non-overlapping manner (to avoid over exposure). Thus, it is known to provide user coaching to assist in the correct use of the IPL device and/or provide automated control of the delivery of light flashes.

The way this is achieved by the system of the invention is described below.

Fig. 2 shows an IPL system comprising an IPL device 10, again with a handle, trigger, light source, light delivery window and controller. The IPL system of Fig. 2 has, for example, the same features as the IPL device of Fig. 1. The controller may perform some of the sensor data processing as well as controlling the light source, and some of the sensor data processing can be performed remotely, for example on an external device such as a smartphone 30 or in the cloud 40. Data can be sent to the external device via wireless communication, such as WiFi or Bluetooth. The smartphone 30 can for example forward the data (optionally after some pre-processing) to a server that runs in the cloud. The results of the calculations are then sent back to the smartphone or the IPL device for displaying them to the user.

The output interface may also be provided at the external device as well as, or instead of, at the hand-held device. Thus, the output interface may comprise the screen (and microphone) of a mobile phone or tablet.

This invention relates to motion tracking of the IPL device 10 and the control of the delivery of flashes of IPL light. Upon start of the treatment, as soon as the skin contact sensor 28 determines that there is contact with the skin surface, the optical displacement sensor 26 starts tracking displacement in two axes, z (which may be defined such that it measures displacement in a direction along the short axis of the treatment window), and x (which may be defined such that it measures displacement in a direction along the long axis of the treatment window).

If the optical displacement sensor 26is mounted at an angle to the treatment window, the measurements can be corrected using the angle by applying trigonometry such that displacements are available along the axes of the treatment window.

For a rectangular treatment window, the movement is typically intended to be along the short axis direction (e.g. z axis) to form a line, and along the long axis direction (e.g. x axis) to move to the path of an adjacent line. By assessing the angle of movement it can be determined if the dominant axis along which the device is being moved is the z axis or the x axis. The correct amount of movement of the sensor in the z axis direction and the x axis direction are different in order to create non-overlapping treatment window areas.

Raw optical displacement measurements are typically available in counts. To convert these raw counts to actual distance (mm) displacement, the counts may be divided by a conversion factor. This factor depends on the optical displacement sensor 26 used and potentially on other factors such as distance to the surface, the surface type (e.g. color and texture), movement speed, and lighting conditions.

In an example, additional sensors may be added to measure these aspects and calibrate the conversion factor accordingly, e.g. using a skin tone sensor and light level sensor. The long term averaged movement sensor data may also be used for calibrating the optical displacement sensor 26.

If skin contact remains, the optical displacement measurements (converted to mm) provide the displacement across the skin in both axes z and x and can be used directly to compute the current position, speed and direction of the device and the timing of the next flash.

In particular, the position at each sampling time instant is obtained by adding the displacement in mm found during the last sampling interval to the previous position (for both axes). The speed can be determined by dividing the displacement in mm found during the last sampling interval by the length of the sampling interval (for both axes). The direction can be determined by comparing the displacements in both axes to each other.

If desired, the angle between the displacements in both axes z and x can be computed to determine an accurate direction. Alternatively, the direction can be limited to the 4 quadrants, e.g., a quadrant with a positive z and positive x displacement, a quadrant with a negative z and positive x displacement, a quadrant with a positive z and negative x displacement, and a quadrant with a negative z and negative x displacement.

The timing of the flash can be determined by recording the last flash location and releasing a flash of IPL light when the device 10 is ready to deliver a flash. The device 10 is ready to flash when the device is charged to deliver the flash, and the distance from all the locations at which all previous flashes took place is at least equal to the treatment window size in the dominant axis direction.

If the device loses skin contact, or is rotated, potentially the new optical displacement locations can no longer be directly compared to previous locations. The movement sensor typically cannot accurately track combinations of rotation and displacement. Hence in one example, the list of previous flash locations can be reset. To determine if the device is rotated, a change in the dominant axis direction can be used to indicate a (potential) rotation of the device. For example, the rotation of the device 10 is determined based on the movement signal.

In another implementation, a flash is only released if the distance from the locations at which all previous flashes were delivered is close to an integer multiple of the treatment window size in the dominant axis direction. This ensures that the user can move backwards to fill-in missed spots without resulting in overlapping flashes if skin contact remains, and the device is not rotated.

To define if the current location is close to a desired flash location, two thresholds can be used, which will be termed gap_threshold, and overlap_threshold.

If the distance to previous locations is smaller than a multiple of (1+gap_threshold)*window_size and larger than a multiple of (1-overlap_threshold)*window_size, the flash can be released. The two thresholds ensure that the amount of overlap and gap remains limited.

As explained above, the reliability of the displacement signal is determined. In one example, this reliability is based on a detected loss of contact, or a contact spacing exceeding a threshold.

If contact with the skin surface is lost, the type of optical displacement sensor will influence whether tracking is still available. For a contact optical displacement sensor (that only measures when there is surface contact), any measurements implemented while contact is lost are unusable. Thus, the displacement signal is not reliable during times of lost contact. The movement sensor output is then used in combination with previous optical displacement measurements to create a best estimate of the displacement during those times. This can rely on the observation that drift of the IMU can be compensated for by using optical displacement and speed measurements while there is surface contact, so that the IMU estimates are more reliable when the contact is lost. If the duration of loss of skin contact is sufficiently short, the quality of the estimate is generally sufficiently high so these estimates can be used to maintain tracking.

For a non-contact optical displacement sensor, the tracking remains valid also if the contact with the skin is lost, as long as the distance is not too large. Thus, the displacement signal is not reliable during times when a contact distance exceeds a threshold. However, generally the displacement measured by the optical displacement sensor is then (linearly) dependent on the distance between the sensor and the skin. If the user loses contact, the distance between the sensor and the skin increases, and therefore the optical displacement measurement underestimates the true distance. To compensate for this effect, a sensor may be included that measures the distance from the skin to the sensor, e.g. a time-of-flight sensor. An IMU sensor may instead be used to estimate the displacement away from the skin from the moment of contact loss. This may be the same sensor as is used for the movement sensing. A distance may be measured based on the combination of contact sensing and acceleration measurements in the direction normal to the treatment window (e.g. the z axis direction).

In these examples, the reliability measure is binary (reliable or not reliable), and one of the first and second contributions is used depending on the binary value. However, the reliability measure may be non-binary, and the first and second contributions may be combined in a more complicated manner.

In one example, an AI model may be used to estimate displacement and also a quality factor from the optical displacement sensor data, the movement sensor data, and the skin contact sensor data (and distance sensor data, if used). In this way, in addition to position, speed, and direction estimates based on the sensor data, a quality factor is also derived. This quality factor indicates how accurate or reliable the estimate is. The quality factor could for example be based on the number of samples for which the contact has been lost (the more lost-contact samples, the lower the quality). Alternatively, the quality factor can be derived using the movement sensor (IMU) data. If a constant speed is kept, the IMU data contains only a gravity component, and little acceleration and rotation is measured. If higher acceleration or rotation is measured, there are larger changes in movement speed, and it becomes more difficult to estimate the current position, speed and direction. Thus, erratic acceleration signals from the movement sensor may be associated with a low quality factor. The quality factor may also take into account detected rotations.

Thus, there may be a reliability measure at least of the displacement sensor output that is used in the computation of the position estimate, and also a quality measure in respect of that estimate.

The accurate position tracking means that flashing can be controlled to take place only at the correct locations, so that over-treatment is prevented while contact with the skin is maintained and the device is not rotated. Furthermore, 100% coverage can be obtained if the user moves the device sufficiently slowly for the device to be ready to flash when the correct spot is reached. If the user moves the device too fast, spots will be missed, and the user may need to move back to fill in the missed spot. If the list of previous flash locations is not reset, this is still feasible, but not missing any spots in the original pass of the device is preferred. Over-treatment may also arise if the remaining untreated gaps are smaller than the relevant treatment window size.

To coach the user to move sufficiently slowly, feedback can be given on the correct speed. Alternatively, the user can be coached to wait at the correct spot for the device to be ready to flash.

The controller 20, for example, produces the timing of the flashes (i.e. when to flash and when not to flash), as well as the direction and speed of movement and current location. Additionally, it can produce information about potential missed spots. Feedback, e.g. about missed spots or moving too fast, can be displayed in a smartphone app, but consumers might not like the idea of having to watch their phone screen all the time during the treatment. Therefore, feedback could also be given via sound coming from the smartphone app (e.g. a computer-generated voice), haptic feedback, or via a visual indicator on the IPL device (e.g. a light indicator or a small display).

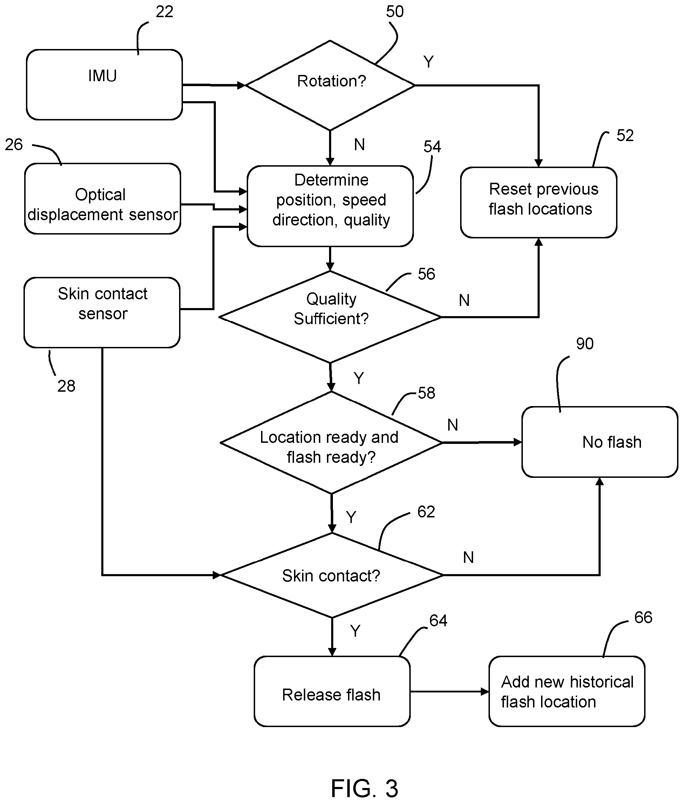

Fig. 3 shows a method of controlling the device 10 in accordance with this disclosure using the concepts described above. It shows the processing of the sensor signals during each sampling window to determine if a flash should be performed in that sampling window.

In step 50, it is determined if there has been a rotation of the device over the skin. This can be derived from the movement sensor (IMU) output.

If there has been a device rotation, the stored flash locations are reset in step 52.

In step 54, the position, speed and direction of the device are determined from the combination of the movement sensor signal, the optical displacement sensor signal and the skin contact sensor signal. In particular, a movement output signal is used for tracking the motion of the device. A first contribution to the movement output signal is made by the displacement sensor 26, and this is the primary contributor to a movement output signal when the displacement signal is reliable, for example while skin contact is maintained. A second contribution to the movement output signal is made by the movement sensor 22 and this is the primary contributor to a movement output signal when the displacement signal is not reliable, for example while skin contact is lost.

The direction is used to determine what the distance between the flashes should be, as this is not (necessarily) the same for the x and y direction in that the light output window is rectangular. The speed is used for coaching the user to maintain a proper slow speed.

The purpose of the movement and displacement analysis is to track the position over time.

Optionally, a quality factor is additionally determined in step 54 that indicates the quality of an estimated position. In step 56, it is determined if the quality factor is sufficient for the position estimate to be relied upon. If not, the stored flash locations are reset in step 52.

If the quality factor is sufficient, it is determined if the flash is ready and if the desired flash location has been reached, in step 58. If not, no flash is yet released (in step 60).

Once the location is reached and the flash is ready, a check is made in step 62 that there is skin contact before releasing a flash in step 64. No flash is released (i.e., step 60) if there is not skin contact.

After release of a flash, the flash location is added to the historical database in step 66.

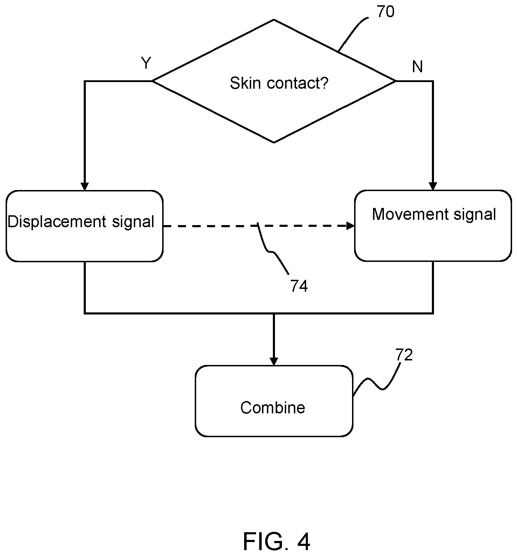

Fig. 4 shows the way movement is tracked in step 54 of Fig. 3. In step 70 skin contact is assessed. Skin contact may be deemed to be made if the distance to the skin is below a threshold, if the motion sensing can be relied upon, or calibrated, up to that distance. If there is skin contact, the optical displacement signal is selected. If there is no skin contact, the movement signal is selected. The selected signals are combined in step 72. The dotted arrow 74 indicates that the optical displacement signal may be used to correct or calibrate the movement signal over time. In particular, drift of the movement sensor is compensated by using displacement and speed measurements made using the displacement sensor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Functions implemented by a controller may be implemented by a single processor or by multiple separate processing units which may together be considered to constitute a "processor". Such processing units may in some cases be remote from each other and communicate with each other in a wired or wireless manner.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A personal care device for application against the skin, comprising:
an optical displacement sensor (26) configured to generate a displacement signal representative of a displacement of the device relative to the skin;
a movement sensor (22) configured to generate a movement signal representative of an absolute movement of the device;
a controller (20) configured to generate a movement output signal representative of a movement of the device,
wherein the controller (20) is configured to:
receive the displacement signal and the movement signal;
use the displacement signal to generate a first contribution to the movement output signal and use the movement signal to generate a second contribution to the movement output signal;
determine a reliability of the displacement signal; and
generate the movement output signal based more, or entirely, on the first contribution when the displacement signal is determined to be reliable and based more, or entirely, on the second contribution when the displacement signal is determined to be not reliable.

2. The device of claim 1, further comprising a skin contact sensor (28) configured to generate a skin contact signal representative of the device being in contact with the skin.

3. The device of claim 2, wherein:
the controller (20) is configured to determine that the displacement signal is not reliable when the device is not in contact with the skin.

4. The device of claim 2, wherein:
the device comprises a skin distance sensor adapted to detect a distance between the device and the skin, and
wherein the controller (20) is configured to determine that the displacement signal is not reliable when the spacing exceeds a threshold.

5. The device of any one of claims 1 to 4, wherein the personal care device comprises an IPL device for delivering IPL light to a user's skin during IPL treatment, and comprises:
an IPL delivery unit (10) comprising:
a light source (16); and
a light delivery window (18);
wherein the controller (20) is configured to control the light source (16) to deliver IPL light to the light delivery window (18) in dependence on the movement output signal.

6. The device of claim 5, wherein the controller (20) is configured to determine a position of the IPL device over time by adding displacements during sensor sampling windows.

7. The device of claim 5 or 6, wherein the controller (20) is configured, when the displacement signal is determined to be reliable, to compensate for drift of the movement sensor by using the displacement signal.

8. The device of any one of claims 5 to 7, wherein the controller (20) comprises an AI model configured to estimate a displacement of the IPL device from a previous location of the IPL delivery unit.

9. The device of claim 8, wherein the controller is configured to generate a quality factor that indicates an accuracy of the displacement estimate, wherein the controller is configured to control the light source to deliver IPL light only when the quality factor exceeds a threshold.

10. The device of any one of claims 5 to 9, wherein the controller (20) is configured to control the light source (16) to deliver IPL light when:
a current position of the IPL delivery unit is at a distance from the positions of a set of previous deliveries of IPL light that is at least equal to a dimension of the light delivery window; or
when a current position of the IPL delivery unit is at a distance from the positions of a set of previous deliveries of light flashes that is approximately equal to an integer multiple of a dimension of the light delivery window.

11. The device of any one of claims 5 to 10, wherein the controller (20) is configured to determine if the IPL delivery unit has been rotated using the movement signal, and if the IPL delivery unit has been rotated, to reset stored locations of previous deliveries of light flashes.

12. The device of any one of claim 5 to 11, further comprising an output interface (24), and wherein the controller is configured to control the output interface based on the movement output signal to provide user feedback in respect of the correct amount of movement between flashes to achieve correct skin coverage.

13. A method of tracking motion of a personal care device that is for application against the skin, comprising:
receiving a displacement signal representative of a displacement of the device relative to the skin;
receiving a movement signal representative of an absolute movement of the device;
(54) generating a movement output signal representative of a movement of the device by:
using the displacement signal to generate a first contribution to a movement output;
using the movement signal to generate a second contribution to the movement output signal;
determining a reliability of the displacement signal; and
generating the movement output signal based more, or entirely, on the first contribution when the displacement signal is determined to be reliable and based more, or entirely, on the second contribution when the displacement signal is determined to be not reliable.

14. A method of controlling IPL device for delivering IPL light to a user's skin during an IPL treatment, comprising:
tracking motion of an IPL delivery unit of the IPL device using the method of claim 13; and
controlling a light source of the IPL device to deliver light flashes to the light delivery window in dependence on the tracked motion of the IPL delivery unit.

15. A computer program comprising computer program code which is adapted, when said program is run on a computer, to implement the method of claim 13 or 14.
